# EUROPEAN PATENT SPECIFICATION

(11) **EP 1 682 191 B1**
(45) Date of publication and mention of the grant of the patent: **18.04.2007**
(21) Application number: 04761980.4
(22) Date of filing: 28.10.2004
(51) Int. Cl.: A61L 9/12

(54) **APPARATUS FOR DIFFUSION OF VOLATILE LIQUIDS**
GERÄT ZUR DIFFUSION VON FLÜCHTIGEN FLÜSSIGKEITEN
APPAREIL POUR LA DIFFUSION DE LIQUIDES VOLATILS

(30) Priority: 10.11.2003 US 518793 P
(43) Date of publication of application: 26.07.2006
(73) Proprietor: Givaudan SA, 1214 Vernier-Genève (CH)
(72) Inventor: MCGEE, Thomas, Nyack, New York 10960 (US); SGARAMELLA, Richard, P., Hoboken, New Jersey 07030 (US); BROWN, Colin, Bracknell Berkshire RG42 2BD (GB); NAISH, Guy Edward, Bicester Oxfordshire OX26 3WE (GB)
(74) Representative: McStea, John Anthony
(86) International application number: PCT/CH2004/000648
(87) International publication number: WO 2005/044321

(56) References cited:
- GB-A- 2 181 649
- US-A- 3 587 968
- US-A- 5 776 561
- US-A1- 2003 146 294

## Description

This invention relates to apparatus for the disseminating of volatile liquids into an atmosphere.

One very common apparatus for disseminating a volatile liquid, such as a fragrance or an insecticide, into an atmosphere consists of a porous transfer member, such as a porous wick, that is in contact with a reservoir of volatile liquid. Liquid rises up this wick and evaporates into the atmosphere. This system has drawbacks, such as the low surface area for evaporation and the tendency for the wick to fractionate complex mixtures, such as fragrances, so that some components are disseminated earlier than others and the full effect of the fragrance is lost.

It has been proposed to overcome this disadvantage by using external capillaries, that is, capillary channels cut or moulded into a suitable substrate. One example is described in United States Patent 4,913,350, in which an external capillary channel-containing member is inserted into a liquid. In another embodiment, described in copending UK Patent Application GB 0306449, there is fitted to a known transfer member a capillary sheet, that is, a sheet extending essentially perpendicularly from the transfer member and that comprises channels of capillary dimensions, to which volatile liquid can pass and travel along for evaporation. This sheet generally contacts the transfer member by means of a hole in the sheet through which the transfer member protrudes and within which it fits snugly, at least some of these channels contacting the transfer member such that liquid can transfer from the member to the sheet ("liquid transfer contact").

Although this technology offers significant advantages over the porous wicks of the art, these advantages have never been completely realized. It has now been found that it is possible to obtain the full benefits of the technology by adherence to certain fundamental parameters. The invention therefore provides an apparatus adapted to disseminate volatile liquid into an atmosphere from a reservoir, the transfer to atmosphere being achieved by means of a porous transfer member that transfers liquid from the reservoir to an evaporation surface, the evaporation surface being a capillary sheet in liquid transfer contact with and extending substantially transversely from the transfer member, and being further characterized in that the material of the sheet is a plastics material having a Shore D hardness of from 50 to 80 and a thickness of from 0.75 -1.25 mm.

The capillary sheet and transfer member are separate components able to be put together when necessary. Typical transfer members include porous wicks of plastics, graphite and ceramics, made by any suitable method, for example, by extrusion or sintering. A preferred material is a porous plastic, such as polyester. The transfer member may be of any suitable shape or construction. Some examples of suitable configurations include;
- the transfer member being slightly frusto-conical in shape, narrower end farthest from the reservoir; this enables a capillary sheet with a circular aperture to be easily slipped on andb fitted to the transfer member;
- the provision in the transfer member of a suitable locating orifice for a capillary sheet, for example, an annular groove or a slot for a matching tab in the capillary diffusion member, to allow for easy fitting.

A combination of all or any of these elements may be used. In addition, other constructions that are not mentioned here but which lie within the skill of the art are also included.

The plastics material may be chosen for any suitable plastics material having the desired hardness properties Shore D hardness is a very well known property in the plastics industry and its measurement is common. It is a surprising feature of this invention that this parameter should have any effect on the efficacy of an evaporation surface, yet it has been found that this has a marked influence. The desired Shore D hardnesses are quite soft by the standards of the plastics industry. Naturally the plastics material should also be suitably inert with respect to any of the materials in the liquid. It should also be a material that permits the formation of capillary channels by any suitable means, for example, by engraving or injection moulding. Injection moulding is the preferred method of forming a capillary sheet. Capillary channels are typically V-shaped channels whose typical dimensions are 0.1-0.5mm wide, 0.1-0.5mm deep with the "V" angle of the channel being 10-25 degrees. The skilled person will readily appreciate what kind of plastics material is suitable for any use.

In a preferred embodiment of the invention, the plastics materials for use in the making of capillary sheets suitable for use in this invention have a surface energy of from 15-50 dyne/cm. The surface energy of a plastics material is dependent upon its molecular structure and is a measure of the ability of a surface to be wetted. The more inert is a plastics material chemically, the lower is its surface energy. Thus, materials such as polyethylene, polypropylene and PTFE have low surface energies, whereas the plastics with more polar groups have higher surface energies. Preferably the surface energy lies in the range of from 30-45 dynes/cm.

The apparatus is generally supplied with the capillary sheet dismounted and a protective cap over the porous member. The apparatus is put into service by removing the cap and placing the capillary sheet on the transfer member, such that it is in liquid transfer contact with the transfer member. This can be assured by use, for example, of one of the attachment means hereinabove described.

The invention is suitable for use with any volatile liquid that may be disseminated into an atmosphere by means of a porous wick. Commercial materials of this type, especially the fragrances, are generally carefully formulated proprietary compositions containing a plurality of ingredients, the precise nature of such compositions being kept confidential. However, whatever the nature of these compositions, it is a feature of this invention that they are much more effectively disseminated by the apparatus according to this invention than by a wick alone.

The invention therefore additionally provides a method of disseminating a volatile liquid into an atmosphere by means of its absorption in and travel along an essentially cylindrical porous wick and then along an evaporation surface extending substantially transversely from the wick and in liquid transfer contact therewith, the evaporation surface comprising a capillary sheet of a plastics material having a Shore D hardness of from 50-80 and a thickness of from 0.75 -1.25 mm.

The invention is further described with reference to the following non-limiting examples. Example 1.

1.00 mm thick external capillary sheets were prepared from the materials in the following table and the Shore hardness measured. This was done by means of a Shore (Durometer) test according to ASTM D2240 00. The apparatus used used was a Mitutoyo Hardmatic HH-337-01. An indenter is pressed in the plastic and this forces the needle of the gauge round and the indicator records maximum value. This is the Shore D Hardness. The individual sheets comprised central holes that permitted them to be mounted on fiusto-conical wicks by simply placing them on the wicks.

| Trade Name | Material Type | Hardness |
|---|---|---|
| | | (Measured) |
| | | (Shore D) |
| BOREALIS* MG 9641-R | Polyethylene PE (HDPE) | 56 |
| SAN* 386R / 774 | Styrene Acrylonitrile Copolymer | 87 |
| | SAN | |
| EXACT* 8210 | Octene-1 Plastomer | 44 |
| POLYAC* PA-758 | Acrylonitrile-butadiene-styrene | 81 |
| | ABS | |
| IUPITAL* F40-03 | Polyoxymethylene POM | 80 |
| | (Acetal) | |
| LUPOY* GP5001 A-F | PC/ABS | 80 |
| IPETHENE* 320 | Polyethylene PE (LDPE) | 45 |
| RADIATER* E AX1 100 | Polyethylene terepthalate | 79 |
| | PETG | |
| LL6201 | Polyethylene PE (LLDPE) | 47 |
| ESCORENE* VL02020 | Ethylene Vinyl Acetate EVA | 41 |
| PS 146L | Polystyrene PS | 83 |
| IUPILON* S3000 | Polycarbonate PC | 84 |
| PP 7075 L1 | Polypropylene PP | 55 |
| ITOCHU* H2O2 | Polyethylene terepthalate | 74 |
| | PETG | |
| IOTEK* 8020 | Ionomer (sodium) | 62 |

| | | |
|---|---|---|
| * Trade marks | | |

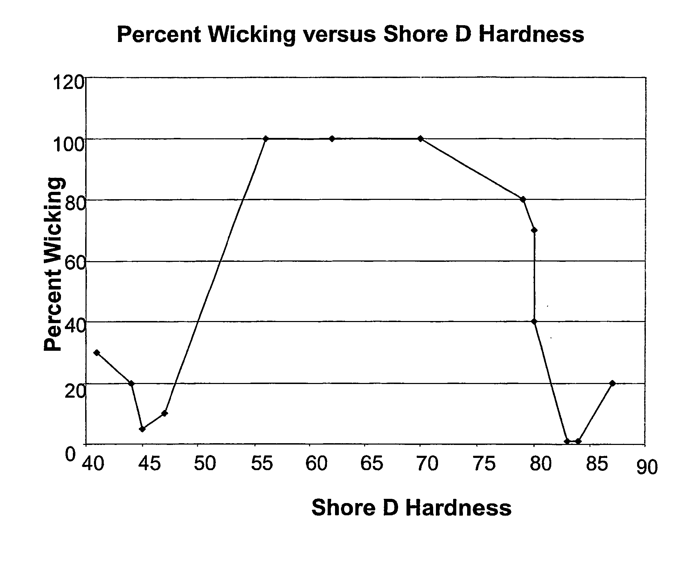

Frusto-conical wicks of polyester (ex Micropore) were placed in a container containing a vanilla fragrance formulation used for air freshening applications (this fragrance has the advantage of being easily visible). They were allowed to equilibrate overnight. The external capillary sheets were pushed on to a frusto-conical wick until firm contact between sheet and wick (suitable to ensure liquid transfer) was made. The percentage of the capillaries in which liquid was present was assessed visually assessed after 6 minutes (0% for no transfer from wick to the capillary, 100% for the presence of liquid in all capillaries and liquid has travelled to end of all capillaries). The results are shown on the graph of Figure 1.

The graph shows that, for optimum liquid transfer, the material has to have a Shore D hardness of between 50 and 80.

### Example 2.

Two frusto-conical wicks were placed in individual containers containing the fragrance formulation of Example 1. They were allowed to equilibrate overnight. To one of these wicks was added a polypropylene capillary sheet having a thickness of 1mm and a Shore hardness of 55, to the other an otherwise identical sheet having a thickness of 1.5mm. The percentage of each sheet wetted by the liquid is shown in the following table.

| Thickness of External Capillary Sheet | % capillary sheet wetted |
|---|---|
| 1.5 mm | 25% |
| 1.0 mm | 100% |

Hence the capillary thickness specification for direct liquid transfer as described in US 4,913,350 is not suitable for use in a hybrid wick system as it does not give optimum liquid transfer contact. The ideal thickness is 1.25 mm maximum. The lower limit of thickness is 0.75 mm set by the limit that good capillaries formed in a suitable production process such as injection molding.

## Claims

1. An apparatus adapted to disseminate volatile liquid into an atmosphere from a reservoir, the transfer to atmosphere being achieved by means of a porous transfer member that transfers liquid from the reservoir to an evaporation surface, the evaporation surface being a capillary sheet in liquid transfer contact with and extending substantially transversely from the transfer member, and being further **characterized in that** the material of the sheet is a plastics material having a Shore D hardness of from around 50 to 80 and a thickness of from 0.75 -1.25 mm.

2. An apparatus according to claim 1, in which the arrangement of transfer member and capillary sheet is selected from the group consisting of:
(i) a transfer member that is slightly frusto-conical in shape, narrower end farthest from the reservoir, on to which a capillary sheet having a circular aperture is slipped and fitted;
(ii) a transfer member comprising a suitable locating orifice into which a capillary sheet may be fitted.

3. An apparatus according to claim 1, in which the plastics materials of the capillary sheet has a surface energy of from 15-50 dyne/cm.

4. An apparatus according to claim 2, in which the surface energy lies in the range of from 30-45 dynes/cm.

5. An apparatus according to claim 1, in which the capillary channels are V-shaped channels that are from 0.1-0.5mm wide, from 0.1-0.5mm deep, and that have a "V" angle of the channel of from 10-25 degrees.

6. A method of disseminating a volatile liquid into an atmosphere by means of its absorption in and travel along an essentially cylindrical porous wick and then along an evaporation surface extending substantially transversely from the wick and in liquid transfer contact therewith, the evaporation surface comprising a capillary sheet of a plastics material having a Shore D hardness of from 50-80 and a thickness of from 0.75 -1.25 mm.

## Patentansprüche

1. Gerät zur Dissemination einer flüchtigen Flüssigkeit in eine Atmosphäre aus einem Reservoir, wobei die Übertragung in die Atmosphäre mittels eines porösen Übertragungselements erreicht wird, das Flüssigkeit aus dem Reservoir zu einer Verdampfungsfläche überträgt, die ein kapillarer Flächenkörper ist, der in Flüssigkeitsübertragungskontakt mit dem Übertragungselement ist und sich im Wesentlichen quer von diesem erstreckt und weiterhin **dadurch gekennzeichnet ist, dass** das Material des Flächenkörpers ein Plastikmaterial ist, das eine Shore-D-Härte zwischen etwa 50 und 80 und eine Dicke zwischen 0,75 und 1,25 mm hat.

2. Gerät nach Anspruch 1, bei dem die Anordnung aus Übertragungselement und kapillarem Flächenkörper aus der Gruppe ausgewählt ist, die besteht aus:
(i) einem Übertragungselement, das leicht kegelstumpfförmiger Gestalt ist, dessen schmaleres Ende vom Reservoir am fernsten ist und auf den ein kapillarer Flächenkörper, der eine kreisförmige Öffnung hat, aufgeschoben und befestigt ist; und
(ii) einem Übertragungselement, das eine geeignete Aufnahmeöffnung hat, in der ein kapillares Flächenelement befestigt werden kann.

3. Gerät nach Anspruch 1, bei dem das Plastikmaterial des kapillaren Flächenelements eine Oberflächenenergie von 15 bis 50 Dyn/cm hat.

4. Gerät nach Anspruch 2, bei dem die Oberflächenenergie im bereich zwischen 30 und 45 Dyn/cm liegt.

5. Gerät nach Anspruch 1, bei dem die Kapillarkanäle V-förmige Kanäle sind, die zwischen 0,1 und 0,5 mm breit und von 0,1 bis 0,5 mm tief sind und einen "V"-Winkel des Kanals von 10° bis 25° haben.

6. Verfahren zur Dissemination einer flüchtigen Flüssigkeit in eine Atmosphäre durch ihre Absorption in einen und ihr Fortschreiten längs eines im Wesentlichen zylindrischen, porösen Dochts und dann längs einer Verdampfungsfläche, die sich im Wesentlichen quer von dem Docht erstreckt und in Flüssigkeitsübertragungskontakt damit ist, wobei die Verdampfungsfläche ein kapillares Flächenelement aus Plastikmaterial umfasst, das eine Shore-D-Härte von 50 bis 80 und eine Dicke von 0,75 bis 1,25 mm hat.

## Revendications

1. Appareil agencé pour la diffusion de liquide volatil dans une atmosphère à partir d'un réservoir, le transfert à l'atmosphère étant obtenu au moyen d'un élément de transfert poreux qui transfère le liquide du réservoir à une surface d'évaporation, la surface d'évaporation étant une feuille capillaire en contact de transfert de liquide avec l'élément de transfert et se prolongeant essentiellement transversalement à partir de l'élément de transfert, **caractérisé, en outre, en ce que** le matériau de la feuille est un matériau en matière plastique ayant une dureté Shore D d'environ 50 à 80 et une épaisseur d'environ 0,75 - 1,25 mm.

2. Appareil selon la revendication 1, dans lequel l'agencement de l'élément de transfert et de la feuille capillaire est choisi dans le groupe comprenant :
(i) un élément de transfert qui est légèrement de forme tronconique, à extrémité plus étroite éloignée du réservoir, sur lequel est glissé et adapté une feuille capillaire ayant une ouverture circulaire ;
(ii) un élément de transfert comprenant un orifice de localisation approprié dans lequel peut être adaptée une feuille capillaire.

3. Appareil selon la revendication 1, dans lequel les matériaux en matière plastique de la feuille capillaire ont une énergie de surface de 15-50 dynes/cm.

4. Appareil selon la revendication 2, dans lequel l'énergie de surface se situe dans la gamme de 30-45 dynes/cm.

5. Appareil selon la revendication 1, dans lequel les canaux capillaires sont des canaux en forme de V qui sont d'une largeur de 0,1-0,5 mm, d'une profondeur de 0,1-0,5 mm et qui ont un angle "V" du canal de 10 à 25 degrés.

6. Procédé pour la diffusion d'un liquide volatil dans une atmosphère au moyen de son absorption et se déplaçant le long d'une mèche poreuse essentiellement cylindrique et ensuite le long d'une surface d'évaporation s'étendant essentiellement transversalement à partir de la mèche et en contact de transfert de liquide avec elle, la surface d'évaporation comprenant une feuille capillaire d'une matière plastique ayant une dureté Shore D de 50-80 et une épaisseur de 0,75-1,25 mm.
